# EUROPEAN PATENT SPECIFICATION

(11) **EP 1 272 044 B1**
(45) Date of publication and mention of the grant of the patent: **11.08.2004**
(21) Application number: 01921529.2
(22) Date of filing: 05.04.2001
(51) Int. Cl.: A23D 7/00, A61K 9/107, A23L 1/24, A23L 1/39, A23L 1/38, A23J 3/08, A23J 3/16

(54) **PROTEIN STABILISED EMULSIONS**
PROTEIN-STABILISIERTE EMULSIONEN
EMULSIONS STABILISEES PAR PROTEINE

(30) Priority: 06.04.2000 GB 0008375; 28.12.2000 GB 0031739
(43) Date of publication of application: 08.01.2003
(73) Proprietor: THE HANNAH RESEARCH INSTITUTE, Ayr, Ayrshire KA6 5HL (GB)
(72) Inventor: MUIR, Donald, c/o Hannah Research Institute, Ayrshire KA6 5HL (GB); CONNOR, Shirley, c/o Hannah Research Institute, Ayrshire KA6 5HL (GB); MCGREGOR, Nancy, c/o Hannah Research Institute, Ayrshire KA6 5HL (GB); NAHRAIN, Chanchal, c/o Hannah Research Institute, Ayrshire KA6 5HL (GB)
(74) Representative: Kennedy, David Anthony
(86) International application number: PCT/GB2001/001482
(87) International publication number: WO 2001/076381

(56) References cited:
- EP-A- 0 212 875
- EP-A- 0 702 902
- EP-A- 0 776 657
- EP-A- 0 914 777
- US-A- 4 183 960
- US-A- 4 304 795
- PATENT ABSTRACTS OF JAPAN vol. 1999, no. 09, 30 July 1999 (1999-07-30) & JP 11 098960 A (FUJI OIL CO LTD), 13 April 1999 (1999-04-13)
- PATENT ABSTRACTS OF JAPAN vol. 1997, no. 10, 31 October 1997 (1997-10-31) & JP 09 149772 A (Q P CORP), 10 June 1997 (1997-06-10)
- DICKINSON E: "PROTEINS AT INTERFACES AND IN EMULSIONS. STABILITY, RHEOLOGY AND INTERACTIONS" JOURNAL OF THE CHEMICAL SOCIETY. FARADAY TRANSACTIONS, ROYAL SOCIETY OF CHEMISTRY, CAMBRIDGE, GB, vol. 94, no. 12, 21 June 1998 (1998-06-21), pages 1657-1669, XP000781909 ISSN: 0956-5000

## Description

The present invention relates to protein stabilised. emulsions which are stable at low pH and in simulations of the gastric environment and which can be used in foodstuffs and in the oral administration of bio-active agents, probiotic organisms, and nutrients. The emulsions may also be used to transport drugs, peptides, hormones, vaccines, and gene therapeutics through the upper gastrointestinal tract to the small intestine.

Without doubt, the most convenient route for administering bioactive agents is by oral administration. Oral administration does not require skilled medical personnel or strict sterile conditions, as are required with other modes of administration such as intravenous injection.

However there are inherent problems associated with the oral route of administration, which are well known to the art. In order to be absorbed into the systemic circulation, the administered agent must passes through the gastrointestinal tract to the small intestine where most absorption into the bloodstream takes place. However agents administered in this manner are often rapidly broken down in the upper gastro-intestinal tract. Orally administered material first encounters saliva in the buccal cavity, which is mildly alkaline and contains the digestive enzyme amylase. Thereafter the material passes down the oesophagus to the gut, where it is subject to highly acidic conditions and degradation by powerful digestive enzymes such as pepsin, rennin and lipase. This is particularly problematic with proteins and peptides which are becoming increasingly prevalent as therapeutic and pharmacological agents, but which are rapidly broken down by proteases in the upper gastro-intestinal tract. Finally, before passing into the small intestine the material is subjected to pancreatic fluid, which contains a number of proteases, lipases and carbohydrate degrading enzymes, bile and intestinal fluid.

There have been numerous attempts to protect agents against the hostile conditions of the upper gastro-intestinal tract so as to increase the proportion of the administered agent which passes to the small intestine, that is to increase the bioavailability of orally administered agents. Conventional approaches included administering particular enzyme inhibitors with the agent to reduce the amount of enzymatic degradation. However this approach can detrimentally impair normal gastric functioning. Furthermore altering the acidity of the gut is not desirable as acid conditions are required for digestion, and any disruption of the normal pH can promote inflammation and infection. More recently, elaborate pharmacological systems have been produced, such as sustained release preparations and specialised protective enteric coatings which protect the enclosed agent from the stomach environment. Other, approaches have used liposomes, which are minute phospholipid vesicles and which can be filled with, for example, non-lipid soluble drugs, which are retained until the liposome is disrupted. However these approaches are highly sophisticated and consequently are expensive. There is therefore a need in the art for simple and inexpensive delivery systems which have low toxicity.

Stabilising emulsions for transport through the hostile conditions of the upper gastro-intestinal tract requires the use of complex emulsifying systems which are costly and require extensive toxicological testing during clinical trials to ensure they are safe before use. It is a first aim of the present invention to provide a method for manufacturing emulsions which are stable in simulated gastric environments, for example at low pH and in the presence of enzymes such as are commonly found in the gut, but retain their bio-activity or nutritive value and can be used to transport a variety of agents through the upper gastro-intestinal tract to the small intestine. A linked aim is to make emulsions from food grade materials and readily available processing methods, which are inexpensive, and easy to produce and do not require the extensive toxicological testing which is necessary for synthetic delivery systems.

When developing agents for oral administration it is important that the palatability and 'mouthfeel' of the agent is considered. It is well known that if an agent administered by mouth has an unpleasant taste, poor patient compliance may result. It is therefore a further linked aim of the present invention to provide palatable emulsions which can be used to protect orally administered agents in the upper gastro-intestinal tract and which masks the flavour of said agents.

It is a yet further aim is to provide palatable emulsions for protecting orally administered agents in the upper gastro-intestinal tract, which are stable in the presence of ethanol and can be used to form the base of new compound beverages, food dressings and sauces.

Sauces containing egg and butter are difficult to manufacture as emulsions including these ingredients are destabilised by the process of freezing and thawing. Inevitably this places a serious limitation on their long-term preservation as it is not possible to freeze-store such sauces, and also on their widespread use as an ingredient. For example, examination of the properties of Hollandaise type sauces - both fresh and reconstituted from dry ingredients - has revealed that this defect is a common feature of all sauces currently available in the local retail market. In addition, systematic experiments utilising conventional technology and traditional formulation have failed to achieve a significant improvement in freeze-thaw performance.

The problem appears to be associated with the nature of the interfacial material stabilising the butterfat emulsion. This interfacial material is disrupted by ice crystal formation during the freezing process and, as a result, the emulsion aggregates forming lumps that are both unsightly and detrimental to mouth feel. It would therefore be a advantage to be able to manufacture a sauce based on an ingredient such as egg or butter, which retains the essential sensory character- i.e., buttery flavour and acidity - of traditional sauces such as Hollandaise Sauce but is little changed by the process of freezing and thawing. As a result, new types of sauce could be developed with hitherto unknown stability.

US-A-4 304 795 discloses a process for preparing a semi-solid dressing, wherein a soybean protein solution is used as the emulsifier. The soybean protein may be partially hydrolysed with an acid or enzyme in order to improve the solubility.

JP-A-11 098 960 refers to the production of an emulsifying agent which is stable at low pH, by hydrolysing a grain (wheat, rice, soybean) protein with a protease at pH lower than 2.5.

In the present Application, references to APSET (acid-stable protein stabilised emulsion technology) refer to the process emulsifying an edible fat in a solution of an edible protein or mixture of proteins or a mixture of proteins, phospholopids and phosphoproteins, either in or converted to the cationic form (i.e., with a net positive charge). Emulsions so formed are inherently stable to the process of freezing and thawing, and are thus superior ingredients for a wide range of foodstuffs and beverages including sauces and dressings. Additionally the appearance and mouth feel of the emulsions, which form the base of such foodstuffs, are superior. Other ingredients, typically flavourings or spices may also be added to the emulsion formed using APSET to tailor the aroma and flavour to any specific requirements.

According to a first aspect of the present invention there is provided a method of producing protein stabilised emulsions, the method comprising the steps of decreasing the pH of a protein solution, to convert it to a cationic form, heating the solution until the protein is solubilised, and then adding a lipid.

Preferably in the step where the protein solution is heated, the protein solution is heated to approximately 65°C.

Preferably in the step where the pH value is decreased, the pH value is decreased to between 1.5 and 3.5.

Optionally the protein solution comprises fractionated or partially purified food grade proteins.

Alternatively the protein solution comprised a protein mixture.

Optionally the protein in the solution is soya protein.

Alternatively the protein in the solution is egg white protein.

Alternatively the protein in the solution is egg yolk protein.

Optionally the lipid is of animal origin.

Alternatively the lipid is of vegetable origin.

Alternatively the lipid is of fish origin.

Preferably the fat:protein ratio of the final emulsions lies between 10:1 and 20:1.

Preferably a pre-emulsion is made from the protein solution and lipid by high speed mixing.

Preferably the pre-emulsion is treated with a high efficiency dispersion technique to prevent creaming.

Optionally the high efficiency dispersion technique is a valve homogeniser.

Alternatively the high efficiency dispersion technique is a high shear mixer.

Alternatively the high efficiency dispersion technique is a microfluidiser.

Alternatively the high efficiency dispersion technique is ultrasonification.

Preferably the pH of the solution is lowered by the addition of an acidic solution.

Optionally the acidic solution is hydrochloric acid.

Alternatively the acidic solution is citric acid.

Preferably the pH of the final solution is increased by the addition of an alkali solution.

Preferably the alkali solution is sodium hydroxide.

A sugar may be added.

According to a second aspect of the present invention there is provided an oil in water emulsion which is stable at low pH and in aqueous ethanol, wherein the emulsion is comprised of a lipid stabilised by a protein in a cationic form.

The lipid may contain one or more bioactive compound.

The lipid may contain lipid soluble compounds.

The lipid may contain a nutrient.

The lipid may contain a vitamin.

The lipid may contain a pharmaceutical agent.

The lipid may contain a hormone.

The lipid may contain a vaccine.

The lipid may contain a protein or peptide.

According to a third aspect of the present invention there is provided a water in oil in water emulsion which is stable at low pH and in aqueous ethanol, wherein the emulsion is comprised of a lipid stabilised by a protein in a cationic form wherein the lipid also comprises one or more aqueous inclusions stabilised at the water oil interface by a protein, and wherein the aqueous inclusions contain inserted material.

The inserted material may include water soluble compounds.

The inserted material may include a nutrient.

The inserted material may include a vitamin.

The inserted material may include bacteria.

The inserted material may include a pharmaceutical agent.

The inserted material may include a protein or peptide.

Preferably where the inserted material is bacteria, the lipid contain nutrients which promote bacteria growth.

Preferably the size of the aqueous inclusions is not limited and can be adapted to suit the size of the inserted material.

A soluble hydrocolloid may be added.

A carbohydrate may be added.

According to a fourth aspect of the present invention there is provided a food or beverage comprising protein stabilised emulsions according to the second or third aspect.

According to the fifth aspect of the present invention there is provided acidic, freeze-thaw stable edible sauces or dressings comprising protein stabilised emulsions according to the second or third aspect.

Preferably the protein and lipid are edible.
Figure 1 is a schematic illustration of the various forms of emulsions which may be produced by the described method;
Figure 2 is a graph showing the effect of multiple passes through a Microfluidiser on the particle size distribution of caseinateate stabilised emulsions;
Figure 3 is a schematic depiction of the isoelectric properties of the protein stabilised emulsions,
Figure 4 is a graph showing the effect of pH, heat-treatment and fat:protein ratio on emulsifying efficiency measured by the specific surface-area;
Figure 5 is a graph comparing the emulsifying efficiency for whey protein stabilised emulsions in different acids and for two heat treatments;
Figure 6 is a plot of the stability of protein stabilised emulsions in simulated gastric fluid, for changes in time, fat content and the fat:protein ratio;
Figure 7 is a graph demonstrating the lipolysis of emulsified fat when exposed to simulated ileal juice, and;
Figure 8 is a main effects plot for the effect of changes in pH, fat content, fat:protein ratio and storage temperature on the stability of protein stabilised emulsions over a period of three days.

Initial studies on APSET focused on the use of proteins derived from milk - caseins and whey proteins - for the manufacture of emulsions that were acid-stable and exhibited novel properties. However, the principles of APSET are not limited to the use of milk protein but can be applied to any edible protein provided it is converted into the cationic form (i.e., with a net positive charge). Thus stable emulsions can be formed by dispersing an edible fat in a solution of an edible protein that is or is subsequently converted to the cationic form.

The exact pH at which protein changes from neutral charge (at its iso-electric point) to net positive charge is a function of the amino acid composition of the particular protein and varies from protein to protein. Therefore, the effective pH at which the application of APSET is optimal varies from protein to protein.

Nevertheless, emulsions formed by APSET have common and novel properties that differ only in degree rather than in kind. For example, soya protein, egg white protein and egg yolk protein have all been shown to endow the emulsion with acid stability in ethanol solutions and resistance to exposure to simulated gastric fluid. Thus, emulsions from APSET have applications in the manufacture of novel beverages and alcoholic liqueurs and also as vehicles for orally administrated drugs, nutrients, vitamins and the like.

### Example Method 1

Preparation of soya protein isolate stabilised emulsions was carried out by warming 500ml of distilled water to 70°C and slowly adding soya protein isolate while stirring vigorously. Once dissolved, 3M Hydrochloric Acid was added until the pH was lowered to 1.5, whilst maintaining the temperature at 70C and stirring. Oil and sugar were added and mixed util dissolved. Weight was made up to 1 kilo with warm distilled water. The solution was then treated with a Silverson mixer for 2 minutes at low speed and then treated with a high efficiency dispersion technique, typically a microfluidiser at 10,000 psi for five passes.

### Example Method 2

Egg white protein emulsions were formed as follows: Egg white protein is added to distilled water at room temperature and mixed with a Silverson mixer at high speed for 2 minutes. pH is slowly lowered to 1.5 with Hydrochloric Acid whilst stirring gently. Soya oil and sugar are added and mixed for 2 minutes using a Silverson mixer. The solution is then treated with a high efficiency dispersion technique, typically a microfluidiser at 10,000 psi for five passes.

Table 1 gives examples of basic recipes which can be used in the production of protein stabilised emulsions. Whey protein concentrate, sodium caseinate, soy protein isolate and egg-white protein were used.

**Table 1.**

| **Formulation of emulsions (12 in total)** | | | | | |
|---|---|---|---|---|---|
| Code | ***Protein, %*** | ***Fat, %*** | **pH** | ***Ratio*** | ***Sugar*** |
| 5 | 5 | 25 | 1.5 | 5/1 | 170 |
| 7.5 | 5 | 35.7 | 1.5 | 7.5/1 | 157.5 |
| 10 | 5 | 50 | 1.5 | 10/1 | 145 |
| | | | | | |

Table 2 shows the stability of emulsions manufactured by APSET using a range of protein types at pH 1.5.

The results were obtained by mixing a portion of emulsion with two parts of an aqueous ethanol solution - ranging in concentration from 20 - 100% - and the stability of the mixture assessed by visual examination.

**Table 2.**

| **Ethanol stability at pH 1.5 of emulsions made using the APSET principle but with different protein types.** | | | | | | | | | | |
|---|---|---|---|---|---|---|---|---|---|---|
| | Ethanol | 20% | 30% | 40% | 50% | 60% | 70% | 80% | 90% | 100% |
| *WPC5* | | - | - | - | - | - | - | - | - | - |
| *WPC7.5* | | - | - | - | - | - | - | - | - | - |
| *WPC10* | | - | - | - | - | - | - | - | - | - |
| *CAS5* | | - | - | - | - | - | - | - | - | - |
| *CAS7.5* | | - | - | - | - | - | - | - | - | - |
| *CAS10* | | - | - | - | - | - | - | - | - | - |
| *SOY5* | | - | - | - | - | - | - | - | - | - |
| *SOY7. 5* | | - | - | - | - | - | - | - | - | - |
| *SOY10* | | - | - | - | - | - | - | - | - | - |
| *EGG5* | | - | - | - | - | - | - | - | - | - |
| *EGG7.5* | | - | - | - | - | - | - | - | - | - |
| *EGG10* | | - | - | - | - | - | - | - | - | - |
| | | | | | | | | | | |
| Code: - denotes no evidence of instability; WPC= whey protein concentrate; Cas = sodium caseinate; SOY = soya protein isolate; EGG = egg white protein. | | | | | | | | | | |

Irrespective of the origin of the protein the emulsions were all stable in aqueous ethanol solutions up to a concentration of 66%. The pH levels of three of the above proteins were raised to 2.5 and the ethanol stability test repeated. The results are shown in Table 3. A portion of emulsion was mixed with two parts of an aqueous ethanol solution - ranging in concentration from 20 - 100% - and the stability of the mixture assessed by visual examination.

**Table 3.**

| **Ethanol stability at pH 2.5 of emulsions made using the APSET principle but with different protein types.** | | | | | | | | | | |
|---|---|---|---|---|---|---|---|---|---|---|
| | | 20% | 30% | 40% | 50% | 60% | 70% | 80% | 90% | 100% |
| *WPC5* | | - | - | - | - | - | - | - | - | - |
| *WPC7.5* | | - | - | - | - | - | - | - | - | - |
| *WPC10* | | - | - | - | - | - | - | - | - | - |
| *CAS5* | | - | - | - | - | - | - | - | - | - |
| *CAS7.5* | | - | - | - | - | - | - | - | - | - |
| *CAS10* | | - | - | - | - | - | - | - | - | - |
| *SOY5* | | - | - | - | - | - | - | - | - | - |
| *SOY7. 5* | | - | - | - | - | - | - | - | - | - |
| *SOY10* | | - | - | - | - | - | - | - | - | - |
| Code - denotes no evidence of instability; WPC= whey protein concentrate; Cas = sodium caseinate; SOY = soya protein isolate. | | | | | | | | | | |

It was also found that emulsions prepared by application of APSET were stable in simulated gastric fluid for at least 6 hours.

These results demonstrate that APSET is a generic technology, which can be carried out using any protein in a cationic form. APSET is widely applicable and provided the protein used to stabilise the emulsion has been converted by acidification into a state in which it has a net positive charge, novel functionally will be exhibited.

APSET can also be used for the manufacture of acidic, freeze-thaw stable edible sauces or dressings based on the emulsification of edible fat in a solution of edible protein, either alone or mixed with other food components, in or subsequently converted to a cationic form.

### Example Method 3

A freeze-thaw stable Hollandaise type sauce is produced as follows.
Butterfat (300g), starch (20g) and dried egg yolk (10g) are pre-weighed into separate containers. Water (570g) and glucose syrup (100g) are heated to 40°C. The dried egg yolk is blended using a high speed mixer, maintaining the temperature of the mixture at 40°C. The pH of this mixture is adjusted to a pH value around 3.7 using a solution of citric acid. The blend is heated to 55°C and melted butterfat is blended into the acid solution containing egg-yolk protein using a high speed mixer (typically, a process time of 5 minutes is sufficient to form a stable, coarse pre-emulsion). The pre-emulsion is then homogenised to form a disperse emulsion. A convenient way to carry out this operation is to pass the pre-emulsion 3 times through a microfluidiser at a pressure of 5000 psi and at 55°C. At this stage it is convenient to blend in spices [for example, salt (0.50%) and pepper (0.03%)]. Finally the whole product is heated to 85°C, held at this temperature for 10 mins, with stirring, (to ensure microbiological stability), packed into sterile containers with lids and cooled. Products made using the APSET principle (in this case using egg proteins), have a delicious buttery taste combined with a fresh acid note but show no deterioration in stability after freezing and thawing.

In general, the method involves first the dissolution of a protein in a volume of water, followed by the addition of a suitable acid to lower the pH, heating until the protein is solubilised, adding an oil to form a pre-emulsion and then treating the pre-emulsion with a high efficiency dispersion technique to inhibit creaming. It is preferred that the emulsions have a fat:protein ration which lies between 10:1 and 20:1. The protein must be in a cationic form.

The protein stabilised emulsions produced by the described method have novel properties in that they are stable in pH values below 3.5 and in solutions of aqueous ethanol. Furthermore as the pH is lowered, the ethanol stability of the protein stabilised emulsions increases.

Furthermore there are provided protein stabilised emulsions which are stable in simulated gastric fluid and for short times in human saliva and which destabilise when mixed with simulated ileal fluid. Accordingly the emulsions can be used to afford protection in the upper gastro-intestinal tract to agents included in the oil phase in oil-in-water emulsions or to agents included within the aqueous phase which is within the oil phase in water-in-oil-water emulsions.

There is also provided protein-stabilised emulsions, made from food-grade materials which are safe to use and have commercial value.

The protein stabilised emulsions are palatable, have a pleasant taste and mask the flavour of any inclusions. They may therefore be useful as food ingredients to form the base of new compound beverages, sauces, and food dressings. The emulsions may also be used in alcoholic drinks as they are stable in aqueous ethanol. The protein stabilised emulsions can be used to enhance the palatability of preparations containing bioactive agents, nutrients or otherwise unpalatable material such as medical tinctures and fish oil. It is recognised that in the present invention that further enhancement of the flavour could be achieved by adding sweeteners or flavour and colour compounds.

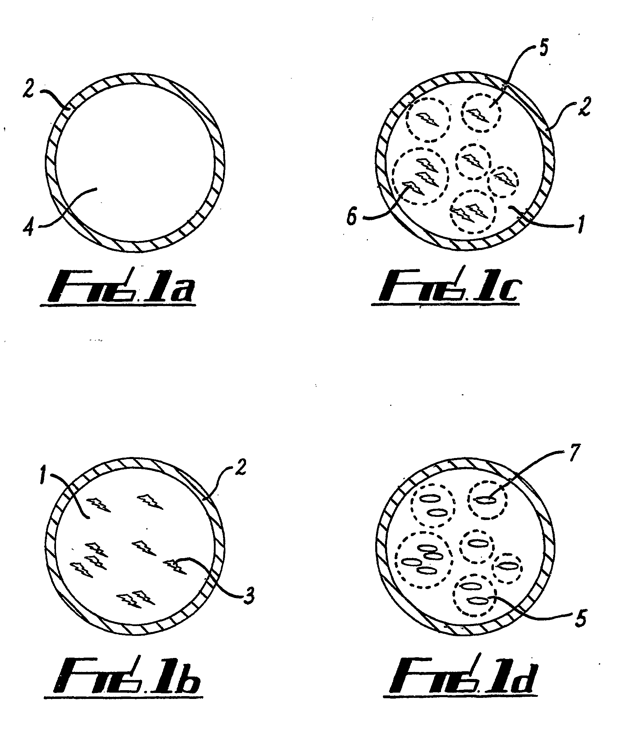

Figure 1 is a schematic representation of the types of emulsions that can be formed by the disclosed method. More particularly Figures 1a and 1b are schematic representations of possible oil-in-water type emulsions, and Figures 1c and 1d are schematic representations of possible water-in-oil-in-water type emulsions. Referring firstly to Figure 1b one possible type of emulsion is oil-in-water which comprises a lipid core of vegetable, animal or fish origin 1, which is stabilised by an interfacial protein 2, in cationic form which could be milk protein such as caseinate or whey protein, soya protein, egg white or egg yolk protein or a combination therefore. The lipid core may contain compounds including but not limited to drugs, nutrients, vitamins, hormones, vaccines and other lipid soluble compounds.
The lipid core may alternatively comprise an oil phase with nutritional value for example fish oil, cod liver oil (Figure 1a). Another type of emulsion is shown in Figure 1c, comprising an interfacial protein 2 which protects a lipid core 1 of animal, vegetable or fish origin, wherein the lipid core has a plurality of aqueous inclusions 5. The aqueous inclusion 5 may contain agents including but not limited to drugs, proteins, gene products and water soluble compounds 6 (Figure 1c), which could not otherwise be transported by the emulsions of 1a and 1b, or bacteria 7 which have useful or beneficial properties (Figure 1d). For example there is a significant body of evidence to suggest that colonisation of the lower digestive tract by certain types of lactic acid bacteria for example Bifidobacteria and Acidophillus spp. has health benefits. These cultures are present in the guts of infants and protect the gut from invasion by other, less desirable bacteria. Normally it is difficult to administer these bacterium, as they are rapidly inactivated by low pH and intestinal fluid and cant be given orally. In the present invention, Lactic acid bacteria can be encapsulated in the protective oil coating of the described emulsions, by including them in internal aqueous inclusions. It is also recognised in the present invention that nutrients which promote bacteria growth can be included in the lipid core of bacteria carrying emulsions. Furthermore, the size of the aqueous inclusions can be adjusted to accommodate the additional matter for example, relatively large bacteria.

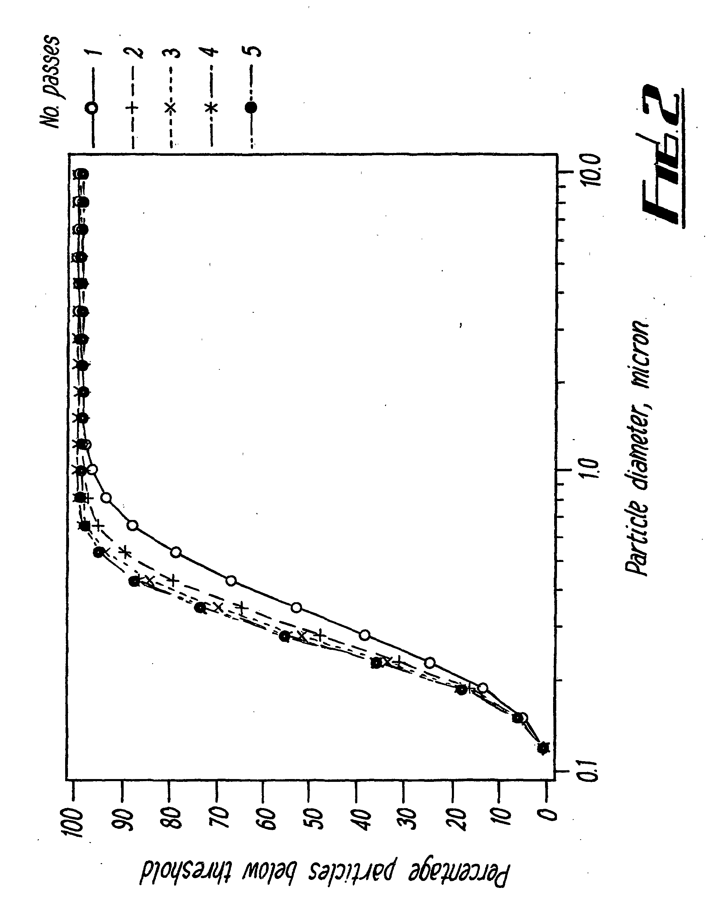

The oil is dispersed in the aqueous phase by emulsification and the newly formed fat surface is stabilised by absorption of protein from the aqueous phase. It will be appreciated to those skilled in the art that it is particularly important that the particle size distribution after emulsification is significantly disperse to avoid creaming. This is achieved by the use of a high efficiency dispersion technique, typically a valve homogeniser, Microfluidiser, high-shear mixer or by ultrasonification. For oil-in-water emulsions creaming is inhibited by reduction of the particle size by repeated treatment in a Microfluidiser, to a range where natural dispersive forces e.g. Brownian Motion) overcome the propensity of creaming. Figure 2 illustrates the particle size and percentage of particles that are below the threshold for creaming after a repeated number of passes through a Microfluidiser. The appropriate particle size depends on the Application. For example, if comparatively large particles, for example bacteria are to be included in the aqueous phase of a water-in-oil-in-water emulsion, the overall size of the protein stabilised globules must be larger to accommodate the inserted material. In this case, creaming is determined by the viscosity of the non-fat phase of the emulsion and may be controlled by the addition of any suitable food ingredient such as hydrocolloid or carbohydrate.

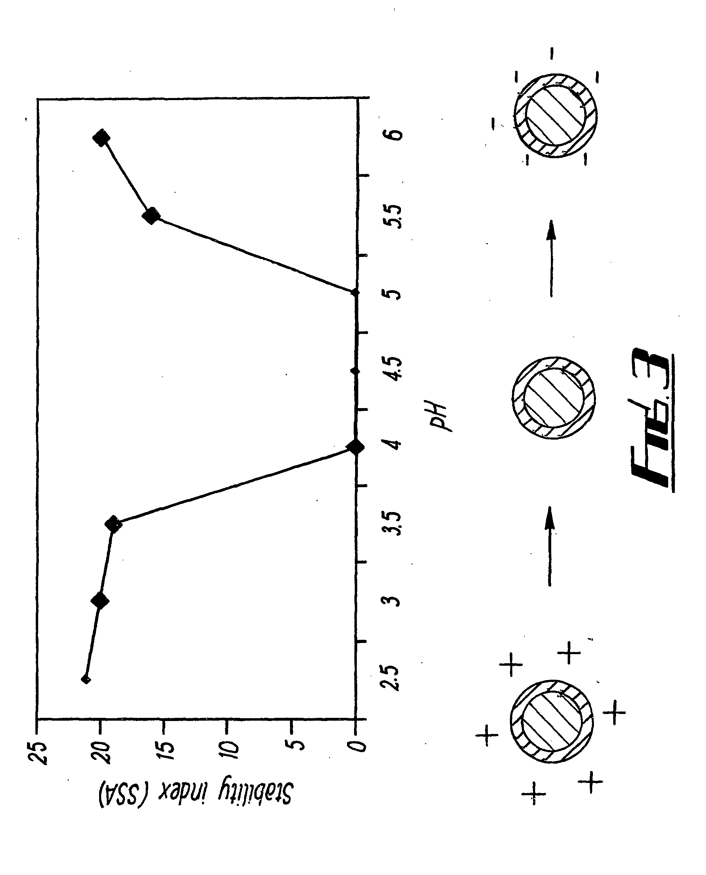

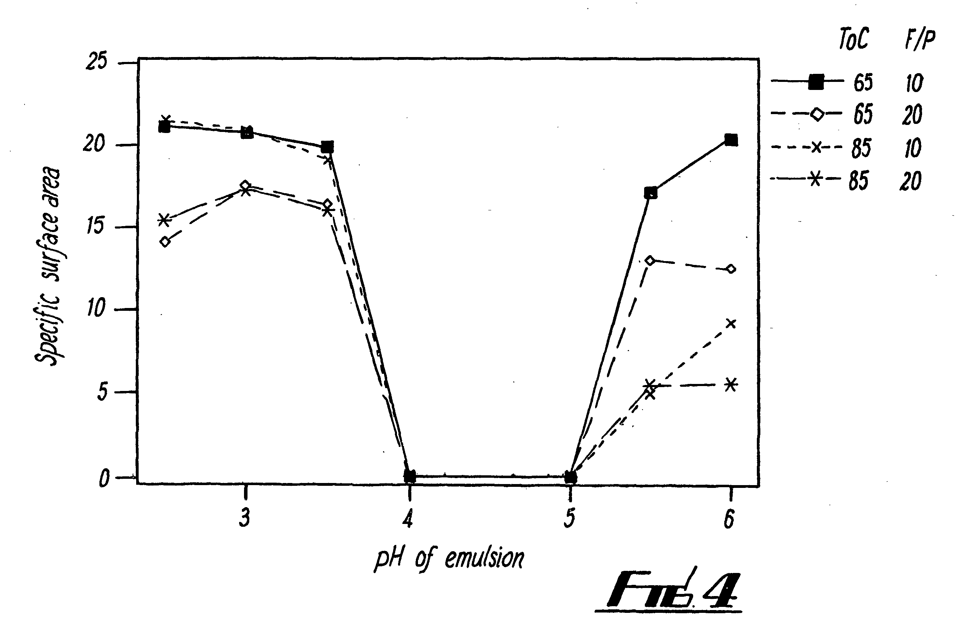

Proteins such as milk proteins are known to have isoelectric points in the range pH 4.5 - 5.0. At neutral pH the proteins are stabilised by a net negative charge as shown schematically in Figure 3. This charge diminishes as the pH is reduced and, by definition, is zero at the isoelectric point, the point where there is no net charge. In the region around the isoelectric point, the isoelectric 'well' the solubility of the protein is reduced and its ability to stabilise fat droplets is severely reduced. Below the isoelectric well there is a positive net charge. The specific surface area (SSA) is a measure of the efficiency of emulsification and a guide to potential long-term stability. Figure 4 shows the SSA of emulsions produced by the described method when subjected to a temperature (ToC) of either 65°C or 85°C and with a fat:protein ratio (F/P) of 10:1 or 20:1. Above the isoelectric well, that is above pH 5.0, higher heat treatment at 85°C reduces the efficiency of emulsification. However, it can be seen from Figure 4 that below the isoelectric well, that is below pH 4.0 the temperature used in the present method is unimportant. The protein stabilised emulsions produced by the described method become more disperse as the pH is lowered. Any temperature between 65°C and 85°C could be used in acidic conditions without reducing the efficiency of emulsification. However it is important that an appropriate fat:protein ratio is used during the described method in order to achieve a finely dispersed emulsion (SSA> 20 m²g⁻¹). That is, protein stabilised emulsions made with a fat:protein ration of 10:1 are significantly more disperse than samples made with a fat:protein ration of 20:1 although any ration between this range typically produces a stable emulsion.

The surprising discovery that protein stabilised emulsions, produced by the method described herein, are stable for considerable lengths of time in ethanol and low pH is an antithesis to the existing body of knowledge in this area (MOHANTY et al.1988; HUNT et al 1994; ABGOOLA et al 1996). Notwithstanding the stability of the protein stabilised emulsions at low pH, the discovery that the emulsions produced by the method described herein are stable in simulated gastric fluid, in the presence of digestive enzymes such a s pepsin and renin was unexpected.

Examples of methods for making emulsions stabilised by whey protein and caseinate are described in depth below.

### Example Method 4

Preparation of whey protein stabilised emulsions (oil in water) was carried out wherein the emulsions contained 50, 100 or 150 gkg⁻¹ fat and had fat:protein rations of 10:1 or 20:1.
Whey protein concentrate (75%) was added to distilled water, warmed to 50°C and dissolved with stirring. The pH of the solution is then adjusted to the required pH value using citric acid solution (0.1M i.e. 19.2gL⁻¹). A suitable oil, in this case a vegetable oil and sugar was added. The addition of a sugar is to maintain a constant solids level and may be omitted from the procedure. A coarse emulsion is then formed using a Silverson high-speed mixer (*ca*. 2 min at 50°C). The coarse emulsion is then heated to either 65°C (equivalent to pasteurisation) or to 85°C (a high heat treatment) for 30 minutes. The emulsion is then cooled to 50°C and treated with a high efficiency dispersion technique; in this case a Microfluidiser at 5 passes at 10,000 psi.

### Example Method 5

Sodium caseinateate stabilised emulsions were obtained as follows.
An appropriate amount of sodium caseinateate was added to 500ml warm (65°C) distilled water to achieve a fat:protein ration in the range 10:1 to 15:1 and vigorously stirred. The pH is then adjusted to 1.5 by the gradual addition of HCl (3.0M), maintaining the temperature at 65°C. Fat and sucrose are then added (typically to yield a fat content of 10-15%). Sucrose can be omitted from the procedure if desired. The mixture is then treated with a high shear Silverston mixer for 2 minutes at 65°C. The final volume was adjusted by addition of distilled water and treated with a high efficiency dispersion technique, in this instance using a Microfluidiser, typically at 5 passes at 10,000 psi.

It is recognised that although examples 5 and 6 have been given for emulsions stabilised by either whey protein or caseinateate, the emulsions may be stabilised by an isolated milk protein, egg white protein, egg yolk protein, soya protein or a mixture of proteins.

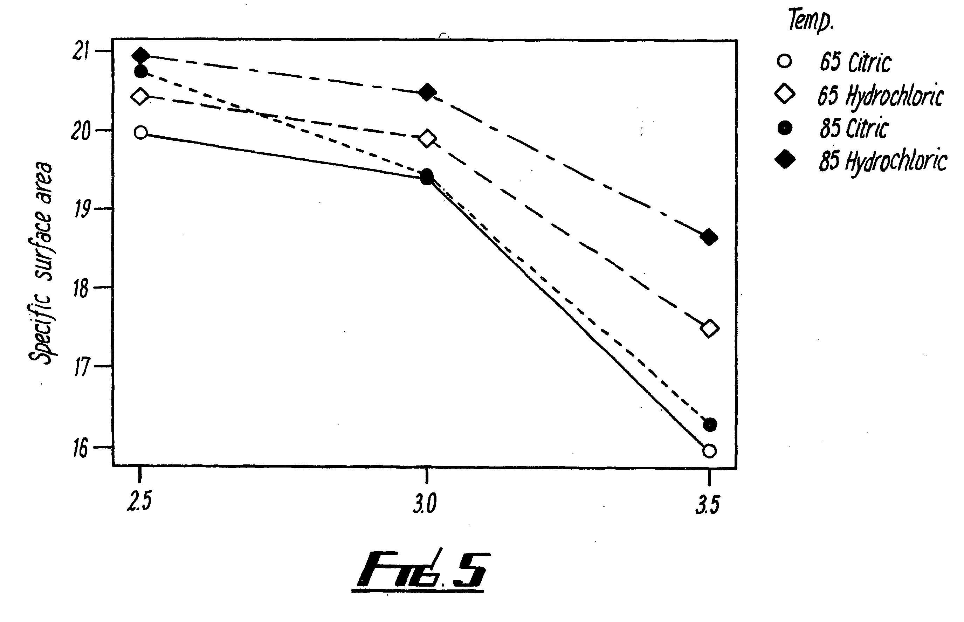

Figure 5 shows that irrespective of the acid which is used to lower the pH of the protein solution, it is the pH used in the method of producing the emulsions which governs the efficiency of the protein stabilised emulsions. However emulsions made by the present method in hydrochloric acid solutions are slightly more disperse than emulsions made in citric acid solutions. In particular there is no significant effect of heating temperature when citric acid is the acidulant, but with hydrochloric acid the higher heat treatment results in a more highly dispersed emulsion.

Previous examples of protein-stabilised emulsions have described the use of fractionated or partially purifed food grade proteins. Mixtures of proteins can also be used successfully. For example, whole milk protein may be used to produce a stable emulsion at acid pH values. There is advantage in carrying out preliminary treatment to reduce the lactose and mineral content as follows. Fat is removed from whole-milk by centrifugal separation at high-speed (standard cream separator, 35° - 68°C). The skim-milk is pasteurized (72°C/15s) to ensure microbial stability then concentrated to half volume by ultrafiltration (hollow fibre membrane, cut off 30,000 Daltons). Distilled water is added to restore the volume and the mixture re-concentrated to half volume. An equal volume of distilled water is added and the volume reduction repeated. The resulting solution is depleted of both carbohydrate and minerals and typically contains >4% true protein. The protein solution is then treated with citric acid to reduce the pH to 2.4. A stable emulsion exhibiting the special characteristics associated with the APSET technique may be made by emulsifying lipid directly into the protein solution using a Microfluidiser or traditional pump homogeniser. The pre-treatment described above is inexpensive and versatile because the protein content and degree of purification may be readily adjusted by changing the ratio of retentate to dilutant and by manipulating the concentration further during ultrafiltration. In addition, because the protein used for emulsification is not dried significant cost savings accrue.

### Properties of the protein stabilised emulsions

It was found that the protein stabilised emulsions produced by this method were not destabilised by short-term exposure to human saliva. The emulsions (pH 1.5) were mixed with saliva, held for 15 seconds and then decanted into simulated gastric fluid with no loss in stability or visual change to the emulsion.

The protein stabilised emulsions were tested for stability in gastric fluid using a simulated gastric fluid. The simulated gastric fluid was prepared by dissolving the following compounds in distilled water and adjusting pH to pH 1.5 using Hydrochloric Acid.

**Table 1**

| **Compounds used to form simulated gastric fluid** | |
|---|---|
| **Compound** | **gL**^{**-1**} |
| proteose peptone | 8.3 |
| d-glucose | 3.5 |
| sodium chloride | 2.05 |
| di-hydrogen potassium phosphate | 0.6 |
| calcium chloride | 0.11 |
| potassium chloride | 0.37 |
| pepsin | 13.3 |
| lysozyme | 0.1 |
| porcine bile | 0.05 |

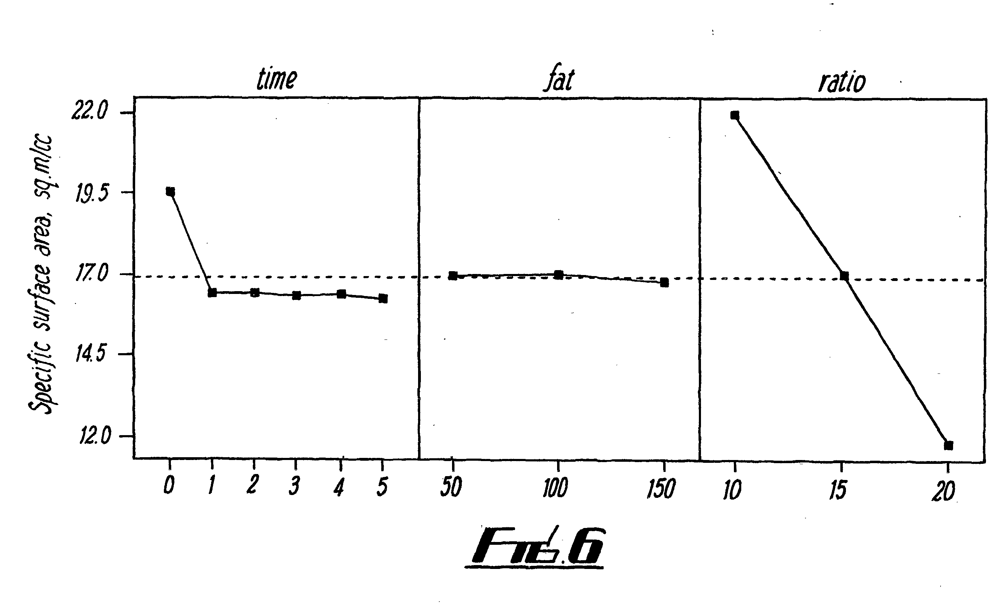

A range of emulsions was manufactured and warmed to 37°C before mixing with the simulated gastric fluid in the ratio 1:4. The mixture was incubated at 37°C for up to 5 hours and particle size distribution was monitored regularly. The main effects over time for up to 5 hours, on fat content, and fat:protein ratio of the emulsion when incubated in simulated gastric fluid are shown in Figure 6. When incubated in the simulated gastric fluid there is a sharp decrease in the Specific surface area within the first hour but very little thereafter. The fat content of the emulsions has little effect on their stability in simulated gastric fluid, however emulsions with a fat:protein ratio of 10:1 are more stable in the simulated gastric fluid than emulsions with a fat:protein ration of 20:1. The latter can also be seen in Figure 4.

At a ratio of 10:1 there is no, or negligible significant change in the emulsion particle size over a 5 hour period. This is important as typically, this is within the range of typical transit times for foods to pass from the stomach to the lower digestive tract.

However, regardless of the stability of the emulsions in the acidic conditions of the stomach, it will be appreciated that in order for a bio active agent to be absorbed into the systemic circulation it will be necessary for the emulsions to be degraded in the small intestine to release the bioactive material contained within. A milk protein stabilised emulsion prepared by the described method was tested in simulated ileal fluid in order to analyse the potential of the emulsion to be degraded in the small intestine. The simulated ileal fluid with a pH of 7 was prepared using the ingredients set out in Table 2:

**Table 2**

| **Ingredients of simulated ileal fluid** | |
|---|---|
| **Substance** | **Quantity** |
| proteose peptide | 5.7gL₋₁ |
| D-Glucose | 2.4 gL⁻¹ |
| NaCl | 6.14gL⁻¹ |
| KH2PO4 | 0.68gL⁻¹ |
| Na_{H2}PO₄ | 0.30gL⁻¹ |
| Na_{HCO3} | 1.01gL⁻¹ |
| Porcine Bile | 11.2gL⁻¹ |
| alpha-amylase | 1000 units/l |
| chymotrypsin | 380 units/l |
| trypsin | 110 units/l |
| lipase | 960 units/l |
| Lysozyme | 0.20gL⁻¹ |

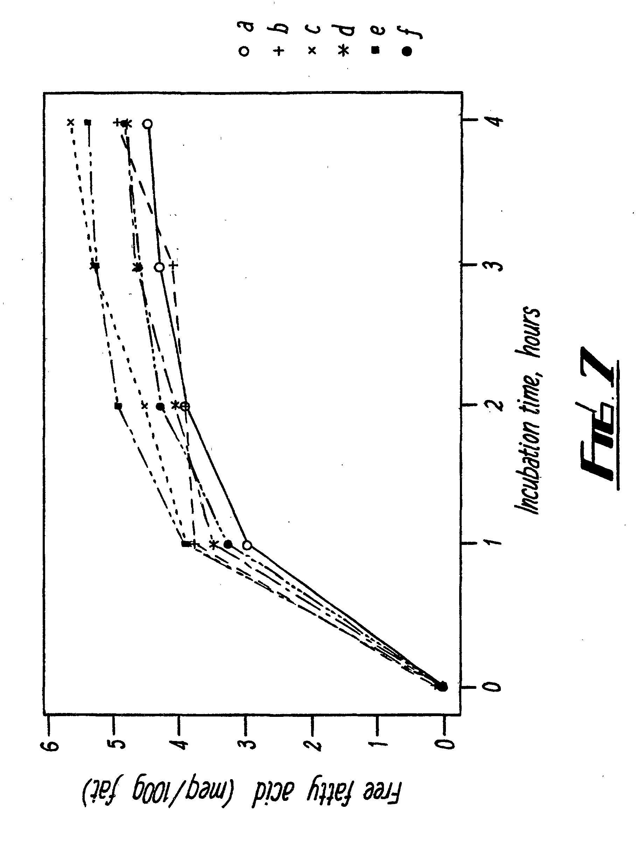

The emulsions were tested in the simulated gastric fluid, as previously described at a 1:4 ratio at pH 1.5, for 3 hours at 37°C. Then portions of the gastric content, including the emulsion, were mixed with the simulated ileal fluid (1:4 ratio pH 7.0) and incubated at 37°C for up to 4 hours. Lipids are usually degraded to free fatty acids by enzymes in the small intestine. Therefore to measure degradation in the small intestine, the free fatty acid content of the mixture was measured at hourly intervals, the results of which are shown in Figure 7. A progressive increase in free fatty acid content was observed.

Therefore the protein stabilised emulsions made by the present method are stable in a simulated gastric environment for up to 5 hours but degrade in conditions close to those in the ileum. More particularly oil-in-water emulsions become susceptible to lipase attack and liberate free fatty acid whereas water-in-oil-in-water emulsions release the encapsulated aqueous insertions as a result of destabilisation of the outer protective protein layer.

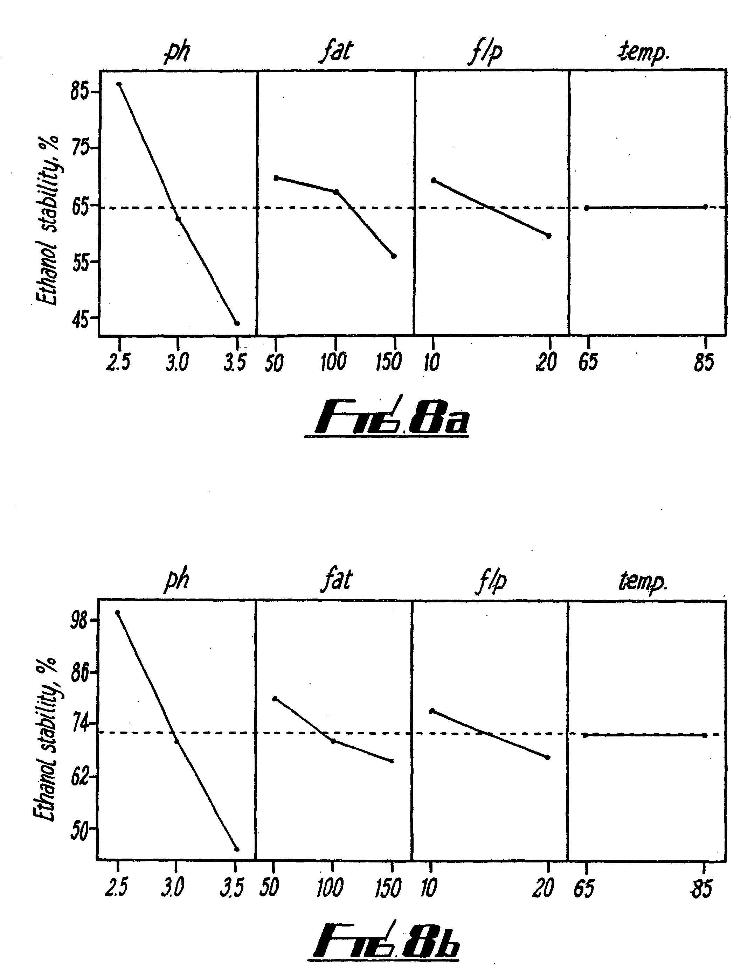

The novel emulsions described here are stable in solutions of aqueous ethanol. Figure 8a is a main effects plot for the effect of changes in pH, fat content, fat:protein ration (f/p) and storage temperature on the stability of the protein stabilised emulsions in ethanol one day after manufacture whilst Figure 8b shows the effects of the aforementioned features on ethanol stability 3 days after manufacture. It can be seen from the Figures 8a and 8b that the pH used when preparing the emulsions is the predominant influence on ethanol stability with modest secondary effects of the fat content and fat:protein ratio of the emulsions. The ethanol stability is also higher after 3 days.

In one form of the invention the emulsions can be used in alcoholic drinks such as cream liqueurs.

### Example Method 7

Sodium caseinateate equivalent to a final concentration of 5% is added to distilled water that is warmed to 65°C, and stirred. 5M of Hydrochloric Acid is then added drop by drop until the protein solution is fully solubilised. The pH is then adjusted back to 2.5 using 2M sodium hydroxide solution and sucrose, equivalent to a final concentration of 10% is added. A pre-emulsion is made using a high shear mixer, and then homogenised using a Microfluidiser (65°C 10,000psi, 5 passes). The emulsion is cooled on ice to below 6°C and ethanol added to a final concentration of 10%. The resulting product has a very strong alcohol content and was found to have no significant deterioration when stored for 21 days at 30°C.

The protein stabilised emulsions and methods for manufacturing protein stabilised emulsions described in the present invention can be used to stabilise sensitive bio-active compounds for example retinol. Inclusion of the compounds in the oil phase ensures a fine dispersion of the bio-active material and aids assimilation. Additionally the emulsions are both microbiologically stable due to the acidity and heat treatment used in their manufacture. In other instances the emulsification process can be carried out at slightly lower temperatures for example 50°C to restrict heat damage to the bioactive compound.

The manufacture of micro-emulsions with a particle droplet size of less than 1 µ is comparatively simple to achieve. Emulsions of this type produced by the APSET process are both physically stable and Brownian motion ensures that creaming takes place very slowly, ie over a period of years. Micro-emulsions are ideal for carrying lipid-soluble material but cannot encapsulate particles of the dimensions associated with bacteria (0.5 - 2 µ). To protect such particles the emulsions must have a droplet diameter in excess of the particle to be protected. Ideally, the particles would have diameters in the range 5 - 20 µ. Stable emulsions of this kind can be made by the APSET principle. For example, macro emulsions can be made in the following way:

Whey protein concentration (WPC 75, 17.5 g) is dissolved in distilled water (50°, 250 g). The pH is adjusted to pH 1.5 using hydrochloric acid. Soya oil (170 g) is blended in by a high speed laboratory mixer (Silverson Machines, Chesham, Bucks) fitted with an emulsifying head. The total mass is adjusted to 500 g by addition of water (at 50°C). Starch (0.1%) is blended in and gelatinised by treatment at 80°C for 15 minutes. The resulting emulsion is transferred to sterile pots, with lids, cooled rapidly to <20°C and stored. The emulsions are stable for at least several weeks at 6°C. A typical particle size distribution is:

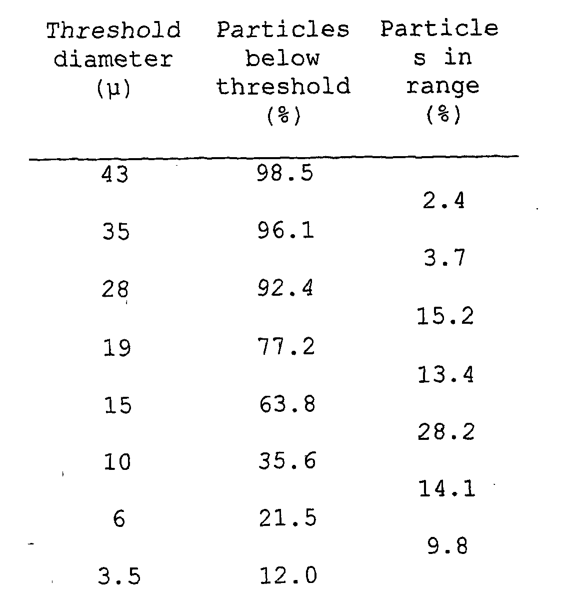

The procedure alone is by way of example only. The starch is not essential and may be replaced by any hydrocolloid or food grade material that is stable in the pH range 1.5 - 4.0 and which increases the viscosity of the emulsion sufficiently to inhibit creaming during extended storage.

The emulsions may also be used to transport a variety of agents including but not limited to bacterium, protein and peptides, hormones, vaccines, gene therapeutics, conventional drugs nutrients and vitamins through the upper gastro-intestinal tract.

Further modifications and improvements may be incorporated without departing from the scope of the invention herein intended.

## Claims

1. A method of producing protein stabilised emulsions, comprising the steps of decreasing the pH of a protein solution, to convert it to a cationic form, heating the solution until the protein is solubilised, and then adding a lipid.

2. A method as claimed in Claim 1, wherein the protein solution is heated to approximately 65°C.

3. A method as claimed in any one of the preceding Claims, wherein the pH value is decreased to between 1.5 and 3.5.

4. A method as claimed in any one of Claims 1-3 wherein the protein solution comprises fractionated or partially purified food grade proteins.

5. A method as claimed in any one of Claims 1-3 wherein the protein solution comprises a protein mixture.

6. A method as claimed in any one of the preceding Claims, wherein the protein in the protein solution is soya protein.

7. A method as claimed in any one of Claims 1 -3, wherein the protein in the protein solution is egg white protein.

8. A method a claimed in any one of Claims 1 - 3, wherein the protein in the protein solution is egg yolk protein.

9. A method as claimed in any one of the preceding Claims, wherein the lipid is of animal origin.

10. A method as claimed in any one of Claims 1 - 8, wherein the lipid is of vegetable origin.

11. A method as claimed in any one of Claims 1 - 8, wherein the lipid is of fish origin.

12. A method as claimed in any one of the preceding Claims, wherein the fat:protein ratio of the final emulsions lies between 10:1 and 20:1.

13. A method as claimed in any one of the preceding Claims, wherein a pre-emulsion is made from the protein solution and lipid by high speed mixing.

14. A method as claimed in Claim 13, wherein the pre-emulsion is treated with a high efficiency dispersion technique to prevent creaming.

15. A method as claimed in Claim 14, wherein the high efficiency dispersion technique is a valve homogeniser.

16. A method as claimed in Claim 14, wherein the high efficiency dispersion technique is a high shear mixer.

17. A method as claimed in Claim 14, wherein the high efficiency dispersion technique is a microfluidiser.

18. A method as claimed in Claim 14, wherein the high efficiency dispersion technique is ultrasonification.

19. A method as claimed in any one of the preceding Claims, wherein the pH of the solution is lowered by the addition of an acidic solution.

20. A method as claimed in Claim 19, wherein the acidic solution is hydrochloric acid.

21. A method as claimed in Claim 19, wherein the acidic solution is citric acid.

22. A method as claimed in any one of the preceding Claims, wherein the pH of the final solution is increased by the addition of an alkali solution.

23. A method as claimed in Claim 22, wherein the alkali solution is sodium hydroxide.

24. A method as claimed in any one of the preceding Claims, wherein a sugar is added.

25. An oil and water emulsion which is stable at low pH and in aqueous ethanol, wherein the emulsion is comprised of a lipid stabilised by a protein in a cationic form.

26. An oil and water emulsion which is stable at low pH and in aqueous ethanol, wherein the emulsion is comprised of a lipid stabilised by a protein in a cationic form obtainable by the method described in Claims 1-24.

27. An oil and water emulsion as claimed in Claim 25-26, wherein the lipid contains one or more bioactive compounds.

28. An oil and water emulsion as claimed in Claims 25 - 26, wherein the lipid contains lipid soluble compounds.

29. An oil and water emulsion as claimed in Claims 25 - 26, wherein the lipid contains a nutrient.

30. An oil and water emulsion as claimed in Claims 25 - 26, wherein the lipid contains a vitamin.

31. An oil and water emulsion as claimed in Claims 25 - 26, wherein the lipid contains a pharmaceutical agent.

32. An oil and water emulsion as claimed in Claims 25 - 26, wherein the lipid contains a hormone.

33. An oil and water emulsion as claimed in Claims 25 - 26, wherein the lipid contains a vaccine.

34. An oil and water emulsion as claimed in Claims 25 - 26, wherein the lipid contains a protein or peptide.

35. A water and oil in water emulsion which is stable at low pH and in aqueous ethanol, wherein the emulsion is comprised of a lipid stabilised by a protein in a cationic form, wherein the lipid also comprises one or more aqueous inclusions stabilised at the water oil interface by a protein, and wherein the aqueous inclusions contain inserted material.

36. A water and oil in water emulsion which is stable at low pH and in aqueous ethanol, wherein the emulsion is comprised of a lipid stabilised by a protein in a cationic form obtainable by the method described in Claims 1-24, wherein the lipid also comprises one or more aqueous inclusions stabilised at the water oil interface by a protein, and wherein the aqueous inclusions contain inserted material.

37. A water in oil in water emulsion as claimed in Claims 35-36, wherein the inserted material includes water soluble compounds.

38. A water in oil in water emulsion as claimed in Claims 35-36, wherein the inserted material includes a nutrient.

39. A water in oil in water emulsion as claimed in Claims 35-36, wherein the inserted material includes a vitamin.

40. A water in oil in water emulsion as claimed in Claims 35-36, wherein the inserted material includes bacteria.

41. A water in oil in water emulsion as claimed in Claims 35-36, wherein the inserted material includes a pharmaceutical agent.

42. A water in oil in water emulsion as claimed in Claims 35-36, wherein the inserted material includes a protein or peptide.

43. A water in oil in water emulsion as claimed in Claim 40, wherein the lipid contains nutri.ents which promote bacterial growth.

44. A water in oil in water emulsion as claimed in Claims 35- 43, wherein the size of the aqueous inclusions is not limited and can be adapted to suit the size of the inserted material.

45. A water in oil in water emulsion as claimed in Claims 35 - 44, wherein a soluble hydrocolloid may be added.

46. A water in oil in water emulsion as claimed in Claims 35 - 45, wherein a carbohydrate is added.

47. A food or beverage comprising protein stabilised emulsions according to Claims 25 - 46.

48. Acidic, freeze-thaw stabile edible sauces or dressings comprising protein stabilised emulsions according to Claims 25 - 46.

## Patentansprüche

1. Verfahren zum Herstellen von Protein-stabilisierten Emulsionen, umfassend die folgenden Schritte: Senken des pH-Wertes einer Proteinlösung, um sie in eine kationische Form umzuwandeln, Erhitzen der Lösung, bis das Protein aufgeschlossen ist, und dann Zusetzen eines Lipids.

2. Verfahren nach Anspruch 1, bei dem die Proteinlösung auf ungefähr 65°C erhitzt wird.

3. Verfahren nach einem der vorhergehenden Ansprüche, bei dem der pH-Wert auf zwischen 1,5 und 3,5 gesenkt wird.

4. Verfahren nach einem der Ansprüche 1 bis 3, bei dem die Proteinlösung fraktioniert oder teilweise gereinigte Proteine von Lebensmittelgüte umfasst.

5. Verfahren nach einem der Ansprüche 1 bis 3, bei dem die Proteinlösung eine Proteinmischung umfasst.

6. Verfahren nach einem der vorhergehenden Ansprüche, bei dem das Protein in der Proteinlösung Sojaprotein ist.

7. Verfahren nach einem der Ansprüche 1 bis 3, bei dem das Protein in der Proteinlösung Eiweiß-Protein ist.

8. Verfahren nach einem der Ansprüche 1 bis 3, bei dem das Protein in der Proteinlösung Eigelb-Protein ist.

9. Verfahren nach einem der vorhergehenden Ansprüche, bei dem das Lipid tierischer Herkunft ist.

10. Verfahren nach einem der Ansprüche 1 bis 8, bei dem das Lipid pflanzlicher Herkunft ist.

11. Verfahren nach einem der Ansprüche 1 bis 8, bei dem das Lipid von Fisch stammt.

12. Verfahren nach einem der vorhergehenden Ansprüche, bei dem das Fett-Protein-Verhältnis der engültigen Emulsionen zwischen 10:1 und 20:1 liegt.

13. Verfahren nach einem der vorhergehenden Ansprüche, bei dem aus der Proteinlösung und dem Lipid durch Hochgeschwindigkeitsmischen eine Voremulsion hergestellt wird.

14. Verfahren nach Anspruch 13, bei dem die Voremulsion mit einer Hochleistungsdispergiertechnik behandelt wird, um Aufrahmen (Creaming) zu verhindern.

15. Verfahren nach Anspruch 14, bei dem die Hochleistungsdispergiertechnik ein Ventil-Homogenisierer ist.

16. Verfahren nach Anspruch 14, bei dem die Hochleistungsdispergiertechnik ein Intensivmischer ist.

17. Verfahren nach Anspruch 14, bei dem die Hochleistungsdispergiertechnik eine Mikroverwirbelungsvorrichtung ist.

18. Verfahren nach Anspruch 14, bei dem die Hochleistungsdispergiertechnik Ultrabeschallung ist.

19. Verfahren nach einem der vorhergehenden Ansprüche, bei dem der pH-Wert der Lösung durch Zusetzen einer sauren Lösung gesenkt wird.

20. Verfahren nach Anspruch 19, bei dem die saure Lösung Chlorwasserstoffsäure ist.

21. Verfahren nach Anspruch 19, bei dem die saure Lösung Zitronensäure ist.

22. Verfahren nach einem der vorhergehenden Ansprüche, bei dem der pH-Wert der endgültigen Lösung durch das Zusetzen einer alkalischen Lösung erhöht wird.

23. Verfahren nach Anspruch 22, bei dem die alkalische Lösung Natriumhydroxid ist.

24. Verfahren nach einem der vorhergehenden Ansprüche, bei dem ein Zucker hinzugefügt wird.

25. Öl-Wasser-Emulsion, die bei niedrigem pH-Wert und in wässrigem Ethanol stabil ist, wobei die Emulsion ein von einem Protein in einer kationischen Form stabilisiertes Lipid aufweist.

26. Öl-Wasser-Emulsion, die bei niedrigem pH-Wert und in wässrigem Ethanol stabil ist, wobei die Emulsion ein von einem Protein in einer kationischen Form mit dem in den Ansprüchen 1 bis 24 beschriebenen Verfahren stabilisiertes Lipid aufweist.

27. Öl-Wasser-Emulsion nach Anspruch 25 oder Anspruch 26, bei der das Lipid wenigstens eine biologisch aktive Verbindung enthält.

28. Öl-Wasser-Emulsion nach Anspruch 25 oder Anspruch 26, bei der das Lipid lipidlösliche Verbindungen enthält.

29. Öl-Wasser-Emulsion nach Anspruch 25 oder Anspruch 26, bei der das Lipid einen Nährstoff enthält.

30. Öl-Wasser-Emulsion nach Anspruch 25 oder Anspruch 26, bei der das Lipid ein Vitamin enthält.

31. Öl-Wasser-Emulsion nach Anspruch 25 oder Anspruch 26, bei der das Lipid ein pharmazeutisches Agens enthält.

32. Öl-Wasser-Emulsion nach Anspruch 25 oder Anspruch 26, bei der das Lipid ein Hormon enthält.

33. Öl-Wasser-Emulsion nach Anspruch 25 oder Anspruch 26, bei der das Lipid einen Impfstoff enthält.

34. Öl-Wasser-Emulsion nach Anspruch 25 oder Anspruch 26, bei der das Lipid ein Protein oder Peptid enthält.

35. Wasser-/Öl-in-Wasser-Emulsion, die bei niedrigem pH-Wert und in wässrigem Ethanol stabil ist, wobei die Emulsion ein das von einem Protein in einer kationischen Form stabilisiertes Lipid aufweist, wobei das Lipid auch wenigstens einen an der Wasser-Öl-Grenzfläche von einem Protein stabilisierten wässrigen Einschluss aufweist und wobei die wässrigen Einschlüsse eingesetztes Material enthalten.

36. Wasser-in-Öl-in-Wasser-Emulsion, die bei niedrigem pH-Wert und in wässrigem Ethanol stabil ist, wobei die Emulsion ein das von einem Protein in einer kationischen Form mit dem in den Ansprüchen 1 bis 24 beschriebenen Verfahren stabilisiertes Lipid aufweist, wobei das Lipid auch wenigstens einen an der Wasser-Öl-Grenzfläche von einem Protein stabilisierten wässrigen Einschluss aufweist und wobei die wässrigen Einschlüsse eingesetztes Material enthalten.

37. Wasser-in-Öl-in-Wasser-Emulsion nach Anspruch 35 oder Anspruch 36, bei der das eingesetzte Material wasserlösliche Verbindungen aufweist.

38. Wasser-in-Öl-in-Wasser-Emulsion nach Anspruch 35 oder Anspruch 36, bei der das eingesetzte Material einen Nährstoff aufweist.

39. Wasser-in-Öl-in-Wasser-Emulsion nach Anspruch 35 oder Anspruch 36, bei der das eingesetzte Material ein Vitamin aufweist.

40. Wasser-in-Öl-in-Wasser-Emulsion nach Anspruch 35 oder Anspruch 36, bei der das eingesetzte Material Bakterien aufweist.

41. Wasser-in-Öl-in-Wasser-Emulsion nach Anspruch 35 oder Anspruch 36, bei der das eingesetzte Material ein pharmazeutisches Agens aufweist.

42. Wasser-in-Öl-in-Wasser-Emulsion nach Anspruch 35 oder Anspruch 36, bei der das eingesetzte Material ein Protein oder Peptid aufweist.

43. Wasser-in-Öl-in-Wasser-Emulsion nach Anspruch 40, bei der das Lipid Nährstoffe enthält, die bakterielles Wachstum fördern.

44. Wasser-in-Öl-in-Wasser-Emulsion nach einem der Ansprüche 35 bis 43, bei der die Größe der wässrigen Einschlüsse nicht begrenzt ist und passend zur Größe des eingesetzten Materials angepasst werden kann.

45. Wasser-in-Öl-in-Wasser-Emulsion nach einem der Ansprüche 35 bis 44, bei der ein lösliches Hydrokolloid zugesetzt sein kann.

46. Wasser-in-Öl-in-Wasser-Emulsion nach einem der Ansprüche 35 bis 43, bei der ein Kohlehydrat zugesetzt ist.

47. Nahrungsmittel oder Getränk umfassend Protein-stabilisierte Emulsionen nach einem der Ansprüche 25 bis 46.

48. Säurehaltige, gefrier-/auftaustabile essbare Saucen oder Dressings, umfassend Protein-stabilisierte Emulsionen nach einem der Ansprüche 25 bis 46.

## Revendications

1. Procédé permettant de produire des émulsions stabilisées par protéine, comprenant les étapes de réduction du pH d'une solution protéinique, de conversion de celle-ci en une forme cationique, de chauffage de la solution jusqu'à solubilisation de la protéine, puis d'addition d'un lipide.

2. Procédé suivant la revendication 1, dans lequel la solution protéinique est chauffée à environ 65 °C.

3. Procédé suivant l'une quelconque des revendications précédentes, dans lequel la valeur de pH est réduite à une valeur comprise entre 1,5 et 3,5.

4. Procédé suivant l'une quelconque des revendications 1 à 3, dans lequel la solution protéinique comprend des protéines alimentaires fractionnées ou partiellement purifiées.

5. Procédé suivant l'une quelconque des revendications 1 à 3, dans lequel la solution protéinique comprend un mélange protéinique.

6. Procédé suivant l'une quelconque des revendications précédentes, dans lequel la protéine de la solution protéinique est une protéine du soja.

7. Procédé suivant l'une quelconque des revendications 1 à 3, dans lequel la protéine de la solution protéinique est une protéine du blanc d'oeuf.

8. Procédé suivant l'une quelconque des revendications 1 à 3, dans lequel la protéine de la solution protéinique est une protéine du jaune d'oeuf.

9. Procédé suivant l'une quelconque des revendications précédentes, dans lequel le lipide est d'origine animale.

10. Procédé suivant l'une quelconque des revendications 1 à 8, dans lequel le lipide est d'origine végétale.

11. Procédé suivant l'une quelconque des revendications 1 à 8, dans lequel le lipide provient d'un poisson.

12. Procédé suivant l'une quelconque des revendications précédentes, dans lequel le rapport graisses/protéines des émulsions finales est compris entre 10:1 et 20:1.

13. Procédé suivant l'une quelconque des revendications précédentes, dans lequel une pré-émulsion est réalisée à partir de la solution protéinique et du lipide par mélange rapide.

14. Procédé suivant la revendication 13, dans lequel la pré-émulsion est traitée avec une technique de dispersion à haut rendement pour empêcher le crémage.

15. Procédé suivant la revendication 14, dans lequel la technique de dispersion à haut rendement est un homogénéisateur à clapet.

16. Procédé suivant la revendication 14, dans lequel la technique de dispersion à haut rendement est un mélangeur à haut cisaillement.

17. Procédé suivant la revendication 14, dans lequel la technique de dispersion à haut rendement est un microfluidiseur.

18. Procédé suivant la revendication 14, dans lequel la technique de dispersion à haut rendement est l'ultrasonification.

19. Procédé suivant l'une quelconque des revendications précédentes, dans lequel le pH de la solution est réduit par l'addition d'une solution acide.

20. Procédé suivant la revendication 19, dans lequel la solution acide est l'acide chlorhydrique.

21. Procédé suivant la revendication 19, dans lequel la solution acide est l'acide citrique.

22. Procédé suivant l'une quelconque des revendications précédentes, dans lequel le pH de la solution finale est accru par l'addition d'une solution alcali.

23. Procédé suivant la revendication 22, dans lequel la solution alcali est la soude caustique.

24. Procédé suivant l'une quelconque des revendications précédentes, dans lequel un sucre est ajouté.

25. Emulsion d'huile et d'eau qui est stable à un pH faible et dans un éthanol-eau, dans laquelle l'émulsion est composée d'un lipide stabilisé par une protéine sous une forme cationique.

26. Emulsion d'huile et d'eau qui est stable à un pH faible et dans un éthanol-eau, dans laquelle l'émulsion est composée d'un lipide stabilisé par une protéine sous une forme cationique par le procédé suivant l'une quelconque des revendications 1 à 24.

27. Emulsion d'huile et d'eau suivant l'une quelconque des revendications 25 et 26, dans laquelle le lipide contient un ou plusieurs composés bioactifs.

28. Emulsion d'huile et d'eau suivant l'une quelconque des revendications 25 et 26, dans laquelle le lipide contient des composés liposolubles.

29. Emulsion d'huile et d'eau suivant l'une quelconque des revendications 25 et 26, dans laquelle le lipide contient un nutriment.

30. Emulsion d'huile et d'eau suivant l'une quelconque des revendications 25 et 26, dans laquelle le lipide contient une vitamine.

31. Emulsion d'huile et d'eau suivant l'une quelconque des revendications 25 et 26, dans laquelle le lipide contient un agent pharmaceutique.

32. Emulsion d'huile et d'eau suivant l'une quelconque des revendications 25 et 26, dans laquelle le lipide contient une hormone.

33. Emulsion d'huile et d'eau suivant l'une quelconque des revendications 25 et 26, dans laquelle le lipide contient un vaccin.

34. Emulsion d'huile et d'eau suivant l'une quelconque des revendications 25 et 26, dans laquelle le lipide contient une protéine ou un peptide.

35. Emulsion d'eau et d'huile dans l'eau qui est stable à un pH faible et dans un éthanol-eau, dans laquelle l'émulsion comprend un lipide stabilisé par une protéine sous une forme cationique, dans laquelle le lipide comprend également une ou plusieurs inclusions aqueuses stabilisées à l'interface eau-huile par une protéine, et dans laquelle les inclusions aqueuses contiennent une substance introduite.

36. Emulsion d'eau et d'huile dans l'eau qui est stable à un pH faible et dans un éthanol-eau, dans laquelle l'émulsion comprend un lipide stabilisé par une protéine sous une forme cationique formée par le procédé suivant l'une quelconque des revendications 1 à 24, dans laquelle le lipide comprend également une ou plusieurs inclusions aqueuses stabilisées à l'interface eau-huile par une protéine, et dans laquelle les inclusions aqueuses contiennent une substance introduite.

37. Emulsion d'eau et d'huile dans l'eau suivant l'une quelconque des revendications 35 et 36, dans laquelle la substance introduite comprend des composés hydrosolubles.

38. Emulsion d'eau et d'huile dans l'eau suivant l'une quelconque des revendications 35 et 36, dans laquelle la substance introduite comprend un nutriment.

39. Emulsion d'eau et d'huile dans l'eau suivant l'une quelconque des revendications 35 et 36, dans laquelle la substance introduite comprend une vitamine.

40. Emulsion d'eau et d'huile dans l'eau suivant l'une quelconque des revendications 35 et 36, dans laquelle la substance introduite comprend des bactéries.

41. Emulsion d'eau et d'huile dans l'eau suivant l'une quelconque des revendications 35 et 36, dans laquelle la substance introduite comprend un agent pharmaceutique.

42. Emulsion d'eau et d'huile dans l'eau suivant l'une quelconque des revendications 35 et 36, dans laquelle la substance introduite comprend une protéine ou un peptide.

43. Emulsion d'eau et d'huile dans l'eau suivant la revendication 40, dans laquelle le lipide contient des nutriments qui favorisent le développement bactérien.

44. Emulsion d'eau et d'huile dans l'eau suivant l'une quelconque des revendications 35 à 43, dans laquelle la taille des inclusions aqueuses n'est pas limitée et peut être adaptée en fonction de la taille de la substance introduite.

45. Emulsion d'eau et d'huile dans l'eau suivant l'une quelconque des revendications 35 à 44, dans laquelle un hydrocolloïde soluble peut être ajouté.

46. Emulsion d'eau et d'huile dans l'eau suivant l'une quelconque des revendications 35 à 45, dans laquelle un hydrate de carbone est ajouté.

47. Aliment ou boisson comprenant des émulsions stabilisées par protéine suivant l'une quelconque des revendications 25 à 46.

48. Sauces ou assaisonnements comestibles stables aux alternances de température acides comprenant des émulsions stabilisées par protéine suivant l'une quelconque des revendications 25 à 46.
